# EUROPEAN PATENT SPECIFICATION

(11) **EP 3 508 158 B1**
(45) Date of publication and mention of the grant of the patent: **16.02.2022**
(21) Application number: 17845012.8
(22) Date of filing: 29.06.2017
(51) Int. Cl.: A61B 34/30, A61B 17/00, A61B 34/00, B25J 11/00

(54) **INSTRUMENT SET AND OPERATION INSTRUMENT**
INSTRUMENTENSATZ UND OPERATIONSINSTRUMENT
ENSEMBLE D'INSTRUMENTS ET INSTRUMENT D'OPÉRATION

(30) Priority: 31.08.2016 CN 201610795377
(43) Date of publication of application: 10.07.2019
(73) Proprietor: Shanghai Microport Medbot (Group) Co., Ltd., Shanghai 201203 (CN)
(72) Inventor: HE, Chao, Shanghai 201203 (CN); HE, Yuyuan, Shanghai 201203 (CN); WANG, Changchun, Shanghai 201203 (CN); YUAN, Shuai, Shanghai 201203 (CN)
(74) Representative: Patentship Patentanwaltsgesellschaft mbH
(86) International application number: PCT/CN2017/090721
(87) International publication number: WO 2018/040707

(56) References cited:
- CN-A- 101 732 093
- CN-A- 102 028 548
- CN-A- 102 119 872
- CN-A- 102 488 554
- CN-A- 105 286 999
- CN-A- 105 380 749
- CN-A- 105 455 902
- CN-A- 106 109 019
- KR-B1- 101 458 760
- US-A- 4 392 140
- US-A- 4 396 919
- US-A- 4 580 461
- US-A1- 2006 199 999
- US-A1- 2015 051 619

## Description

### TECHNICAL FIELD

The present invention relates to the technical field of medical instruments, and in particular, to an instrument set and a surgical instrument.

### BACKGROUND

Compared with the conventional open surgery, the minimally invasive surgery has obvious advantages in terms of therapeutic effect, pain relief, recovery cycle, and medical cost, etc., and is performed mainly by virtue of advanced medical instruments and equipment with minor wounds.

Traditional laparoscopic surgery is performed mainly by using manual micro-instruments with less in degree of freedom and unsatisfactory in operating space. It generally requires an operator to make lots of auxiliary movements to achieve a certain operating space, and thus, the difficulty of a surgical operation is raised, the surgical efficiency is reduced, and it also causes unnecessary additional damage to patients. With respect to this problem, with the development of robot technologies and related technologies, the scope of surgery of minimally invasive surgical robots has gradually involved more complicated surgical fields, and the surgical operations have also become intelligent and precise. At present, the Da Vinci surgical robot system developed by Intuitive Surgical Company of the United States and the ZEUS system developed by Computer Motion Company have been successfully introduced to the clinic internationally, but they have shortcomings in terms of complicated mechanism, large size and high price, etc. At present, the research on minimally invasive laparoscopic surgery robots has begun in China, but there is still a big gap compared with foreign countries.

After studying the development of the surgical robot system, it is found that the surgical instruments of the minimally invasive surgical robot plays a crucial role in the design of the entire robot system. Therefore, the performance level of the surgical instrument is also one of the key factors in the performance level of the entire surgical robot system, and the posture adjustment of the surgical instrument is achieved by the instrument set disposed at one end of the surgical instrument. For example, the instrument set disclosed in US Patent No. 8,911,428 B2, an end instrument assembly of the surgical instrument is driven by twelve wire ropes for pitching motion. However, the shortcoming of the solution of the above patent is that the pitching motion of the end instrument assembly is achieved based on twelve wire ropes, which makes the structural design of the instrument set more complicated, and the instrument set takes up a large space. CN 105 455 902 A discloses a surgical robot. US 2006/199999 A1 discloses a minimally invasive surgical instrument. US 2015/051619 A1 discloses force transmissions for use in surgical instruments.

### SUMMARY OF THE INVENTION

An objective of the present invention is to provide an instrument set for solving the problem in the prior art that the instrument set requires twelve wire ropes to realize the pitching motion of the end instrument assembly, resulting in a complicated structural design and a large space occupation of the instrument set.

To solve the foregoing technical problem, the present invention provides an apparatus for controlling movement of a surgical instrument, including:
a pedestal;
an overturning platform assembly disposed above the pedestal, wherein the overturning platform assembly includes an inner rotating member, an outer rotating member, a first rotating shaft, and a second rotating shaft; the outer rotating member is disposed on an outer side of the inner rotating member and is connected to the inner rotating member via the second rotating shaft; the outer rotating member is able to rotate about an axis of
the first rotating shaft, the inner rotating member is able to rotate about an axis of the second rotating shaft, and the axis of the first rotating shaft is intersected with the axis of the second rotating shaft to form a first intersection point;
two sets of driving wires, each set of driving wires including two driving wires;
two wire rotating bodies disposed on the pedestal and configured to drive the driving wires;
each of the two sets of driving wires corresponds to one of the wire rotating bodies; one end of each of the two driving wires in each set of driving wires is fixed on the inner rotating member and forms a fixation point; two fixation points formed by each set of driving wires are symmetrical about the first intersection point; four fixation points formed by the two sets of driving wires on the inner rotating member are sequentially connected to form a first parallelogram, and an intersection point of diagonal lines of the first parallelogram coincides with the first intersection point, and the other end of each of the two driving wires in each set of driving wires is wound on the corresponding wire rotating body in a direction opposite to the fixation direction of the one end of each of the two driving wires in each set of driving wires;
and
   four output wires, wherein one end of each output wire is fixed on the inner rotating member and forms a fixation point, four fixation points formed by the four output wires on the inner rotating member are sequentially connected to form a second parallelogram, and an intersection point of diagonal lines of the second parallelogram coincides with the first intersection point, and the other end of each output wire extends through a first through hole formed on the pedestal.

Alternatively, in the instrument set, an axis of the first rotating shaft and an axis of the second rotating shaft are perpendicular to each other and are intersected at the first intersection point.

Alternatively, in the instrument set, a fixed point formed by one set of driving wires on the inner rotating member is located on the first rotating shaft, and a fixed point formed by the other set of driving wires on the inner rotating member is located on the second rotating shaft.

Alternatively, in the instrument set, the second parallelogram is a rectangle or a square.

Alternatively, the instrument set further includes two guide wheel assemblies, wherein each guide wheel assembly corresponds to a set of driving wires for changing an extension direction of the driving wires.

Alternatively, in the instrument set, each guide wheel assembly includes two guide wheels, each guide wheel corresponds to one driving wire, and each driving wire forms two tangent points with the corresponding guide wheel, wherein the tangent point distant from the overturning platform assembly is a second tangent point, and the tangent point close to the overturning platform assembly is a first tangent point, and a portion of each driving wire between the first tangent point and the fixed point is perpendicular to the pedestal.

Alternatively, the instrument set further includes a guide wheel mounting base disposed on the pedestal, wherein the two guide wheel assemblies are disposed on the guide wheel mounting base.

Alternatively, in the instrument set, a first through hole is formed on the pedestal, and a second through hole is formed on the guide wheel mounting base, and the first through hole and the second through hole are concentric to each other and together constitute an extension channel for the four output wires.

Alternatively, the instrument set further includes a wire splint disposed on the inner rotating member and configured to clamp and secure one end of the driving wire and/or the output wire to the inner rotating member.

Alternatively, the instrument set further includes a first support member and a second support member, wherein the first support member and the second support member are disposed on the pedestal and located on opposite sides of the second rotating shaft, and the first support member and the second support member are rotatably connected to the outer rotating member through the first rotating shaft for supporting the overturning platform assembly.

Alternatively, in the instrument set, the two wire rotating bodies include a first wire rotating body and a second wire rotating body, and the first wire rotating body and the second wire rotating body are disposed on the pedestal and located on opposite sides of the guide wheel mounting base.

Alternatively, in the instrument set, the first wire rotating body is provided with a wire mounting hole for fixing the driving wire on the first wire rotating body, and the second wire rotating body is provided with a wire mounting hole for fixing the driving wire on second wire rotating body.

Alternatively, in the instrument set, the first wire rotating body includes a first driving spindle configured to drive the first wire rotating body to perform a rotation motion, and the second wire rotating body includes a second driving spindle configured to drive the second wire rotating body to perform a rotation motion.

Alternatively, in the instrument set, an axis of the first driving spindle is perpendicular to the pedestal, and an axis of the second driving spindle is perpendicular to the pedestal.

Alternatively, in the instrument set, the driving wires and the output wires are wire ropes.

The present invention provides a surgical instrument, including an instrument set, an instrument tube, and an end instrument assembly, wherein the instrument set is connected to the end instrument assembly through the instrument tube and controls the movement of the end instrument assembly.

Alternatively, in the surgical instrument, the instrument tube has a lumen, and four output wires of the instrument set are connected to the end instrument assembly through the lumen of the instrument tube.

In the instrument set and the surgical instrument provided by the present invention, the instrument set includes an overturning platform assembly, two sets of driving wires, two wire rotating bodies, and four output wires. Each set of driving wires includes two driving wires, one end of each of the two driving wires is fixed on the inner rotating member and forms a fixed point; the two fixed points formed by each set of driving wires are symmetrical about the first intersection point; four fixed points formed by the four driving wires on the inner rotating member can be sequentially connected to form a first parallelogram, and an intersection point of diagonal lines of the first parallelogram is the first intersection point, and the other end of each of the two driving wires in each set of driving wires is wound on the corresponding wire rotating body in the opposite direction, and therefore the overturning platform assembly is driven to move by the four driving wires, and then motion of the overturning platform assembly is transferred to an end instrument assembly through the output wires, so that multi-direction and multi-degree-of-freedom motion of the end instrument assembly is achieved, thereby simplifying the design of the instrument set, reducing the dimension of the instrument set, and increasing the space utilization of the instrument set.

### BRIEF DESCRIPTION OF THE DRAWINGS

FIG. 1 is a schematic structural diagram of a surgical instrument according to an embodiment of the present invention;
FIG. 2 is a schematic structural diagram of an instrument set according to an embodiment of the present invention;
FIG. 3 is a bottom view of an overturning platform assembly in FIG. 2;
FIG. 4 is a schematic structural diagram showing that a driving wire is fixed to the overturning platform assembly according to an embodiment of the present invention;
FIG. 5 is a layout diagram of FIG. 4;
FIG. 6 is a schematic structural diagram showing that the overturning platform assembly is disposed on a pedestal in FIG. 4;
FIG. 7 is a schematic structural diagram showing that the driving wires pass through a guide wheel assembly and are fixed on a wire rotating body in FIG. 6; and
FIG. 8 is a schematic structural diagram showing that an output wire is fixed to the overturning platform assembly according to an embodiment of the present invention;
in the drawings: instrument set 1, first driving spindle 11, first wire rotating body 12, first wire rope 13, second guide wheel 14, second wire rope 15, first guide wheel 16, second driving spindle 17, second wire rotating body 18, third wire rope 19, fourth wire rope 110, third guide wheel 111, fourth guide wheel 112, outer rotating member 113, inner rotating member 114, wire splint 115, fifth wire rope 116, sixth wire rope 117, seventh wire rope 118, eighth wire rope 119, pedestal 120, guide wheel mounting base 121, first rotating shaft 122, second rotating shaft 123, instrument tube 2, and end instrument assembly 3.

### DETAILED DESCRIPTION

The instrument set proposed by the present invention will be further described in detail below with reference to the accompanying drawings and specific embodiments. Advantages and features of the present invention will be apparent from the description and appended claims below. It should be noted that the drawings are in a very simplified form and use non-precise proportions, and are only intended to conveniently and explicitly assist in describing the objectives of embodiments of the present invention.

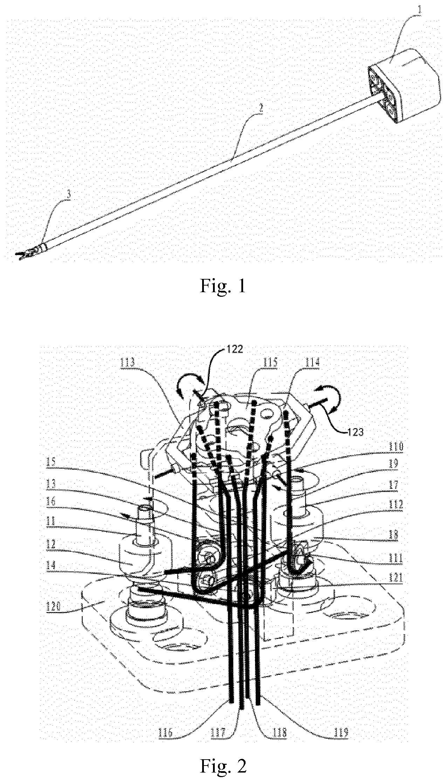

Referring to FIG. 1, which is a schematic structural diagram of a surgical instrument according to the present invention, the surgical instrument includes: an instrument set 1, an instrument tube 2, and an end instrument assembly 3. The instrument set 1 is connected to the end instrument assembly 3 via the instrument tube 2 and controls the movement of the end instrument assembly 3.

Referring to FIG. 2, which is a schematic structural diagram of the instrument set 1 according to the present invention. The instrument set 1 includes a pedestal 120, an overturning platform assembly, two wire rotating bodies, two sets of driving wires, and four output wires, wherein the wire rotating bodies drive the driving wires to move, and further, the movements of the output wires and the end instrument assembly connected to the output wires are controlled via the overturning platform assembly connected to the driving wires. Specifically, the overturning platform assembly includes an inner rotating member 114, an outer rotating member 113, a first rotating shaft 122, and a second rotating shaft 123. The outer rotating member 113 is disposed on the outer side of the inner rotating member 114 and is connected to the inner rotating member 114 via the second rotating shaft 123. The outer rotating member 113 rotates about the first rotating shaft 122. The inner rotating member rotates around the second rotating shaft 123. An axis of the first rotating shaft 122 and an axis of the second rotating shaft 123 are intersected at a first intersection point, preferably vertically intersected at a first intersection point. The first intersection point may be a center point of the inner rotating member 114. Each set of driving wires include two driving wires. Two wire rotating bodies are disposed on the pedestal 120. Each set of driving wires corresponds to one of the wire rotating bodies, and the wire rotating bodies are configured to drive the driving wires. One end of each of the two driving wires in each set of driving wires is fixed on the inner rotating member 114 and forms a fixed point, The two fixed points formed by each set of driving wires are symmetrical about the first intersection point. The four fixed points formed by the two sets of driving wires on the inner rotating member 114 can be sequentially connected to form a first parallelogram, and an intersection point of diagonal lines of the first parallelogram is the first intersection point, and the other end of each of the two driving wires in each set of driving wires is wound on the corresponding wire rotating body in the opposite direction. One end of each of the four output wires is fixed on the inner rotating member 114 and forms a fixed point, four fixed points formed on the inner rotating member 114 are sequentially connected to form a second parallelogram, and an intersection point of diagonal lines of the second parallelogram is the first intersection point, and the other end of each output wire extends toward the pedestal and is connected to the end instrument assembly 3 via a lumen of the instrument tube 2, so as to achieve posture adjustment of the end instrument assembly 3. Preferably, the inner rotating member 114 is an inner rotating plate, and the outer rotating member 113 is an outer rotating frame.

More specifically, in this embodiment, the fixed point formed by one of the two sets of driving wires on the inner rotating member is located on the first rotating shaft, and a fixed point formed by the other set of driving wires on the inner rotating member is located on the second rotating shaft. Four fixed points formed by the four output wires on the inner rotating member 114 are sequentially connected to form a rectangle or a square, and more preferably, a connecting line of two adjacent fixed points among the four fixed points formed by the four output wires on the inner rotating member 114 is parallel to the first rotating shaft or the second rotating shaft.

Further, the instrument set 1 further includes a wire splint 115, two guide wheel assemblies, and a guide wheel mounting base 121. The wire splint 115 is disposed on the inner rotating member 114 and configured to clamp and secure one end of the driving wire and/or the output wire to the inner rotating member 114. The guide wheel mounting base 121 is disposed on the pedestal 120 and configured to change the extension direction of the driving wires. The two guide wheel assemblies are disposed on the guide wheel mounting base. Each guide wheel assembly corresponds to a set of driving wires, and each guide wheel assembly includes two guide wheels. Each guide wheel in each guide wheel assembly corresponds to a driving wire in the corresponding set of driving wires, that is, two driving wires in a set of driving wires are wound on different guide wheels in the same guide wheel assembly. Each driving wire is changed in the extension direction by the corresponding guide wheel and then extends to the wire rotating body corresponding to the set of driving wires. A first tangent point is formed when the driving wire enters the guide wheel, that is, a tangent point of the driving wire close to the overturning platform assembly is a first tangent point. A second tangent point is formed when the driving wire leaves the guide wheel, that is, a tangent point of the driving wire distant from the overturning platform assembly is a second tangent point. And the purpose of changing the extension direction of the driving wires is achieved by distributing four guide wheels at the periphery of the guide wheel mounting base 121. In addition, the guide wheels are arranged such that the portion of the driving wire extending from the inner rotary member 114 to the guide wheel is perpendicular to the guide wheel mounting base 121. At this moment, the portion of each driving wire between the first tangent point and the fixed point is perpendicular to the pedestal. Preferably, each group of the guide wheels can rotate freely to reduce the friction between the guide wheel and the driving wire.

In this embodiment, a first through hole is formed on the pedestal 120, and a second through hole is formed on the guide wheel mounting base 121. The first through hole and the second through hole are concentric to each other, and together constitute an extension channel for the four output wires. The four output wires sequentially pass through the second through hole and the first through hole to enter the instrument tube 2 to be connected to the end instrument assembly 3, and the transfer channels of the four output wires are limited by the second through hole and the first through hole, to avoid the interference of the extension of the four output wires on the driving wire, so that the stability and reliability of the instrument set 1 are improved.

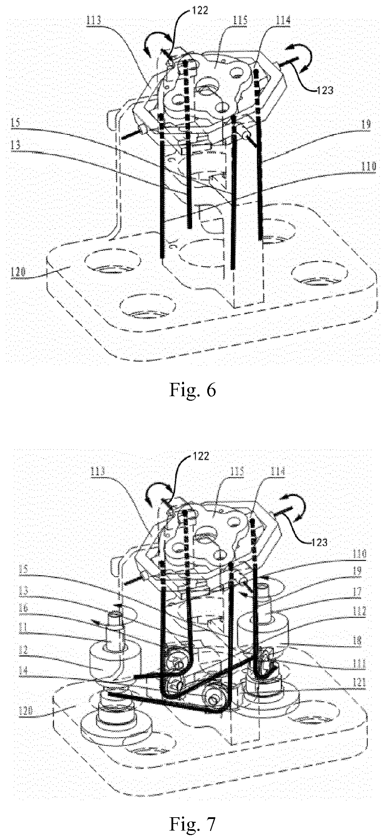

Referring to FIGs. 6 and 7, the instrument set 1 further includes a first support member and a second support member configured to support the overturning platform assembly. The first support member and the second support member are disposed on the pedestal 120 and located on opposite sides of the second rotating shaft 123. The first support member and the second support member are rotatably connected to the outer rotating member 113 via the first rotating shaft 122 for supporting the overturning platform assembly. The axis of the first rotating shaft 122 is concentrically mounted with and in clearance fit to the center of pin holes of the first support portion and the second support portion on the pedestal 120 in detail. The outer rotating member 113 is supported by the first supporting member and the second supporting member, and the inner rotating member 114 inside the outer rotating member 113 is rotated by the driving force of the two sets of driving wires, and thus the end instrument assembly 3 is driven by the four output wires fixed on the inner rotating member to achieve two degrees of freedom motion.

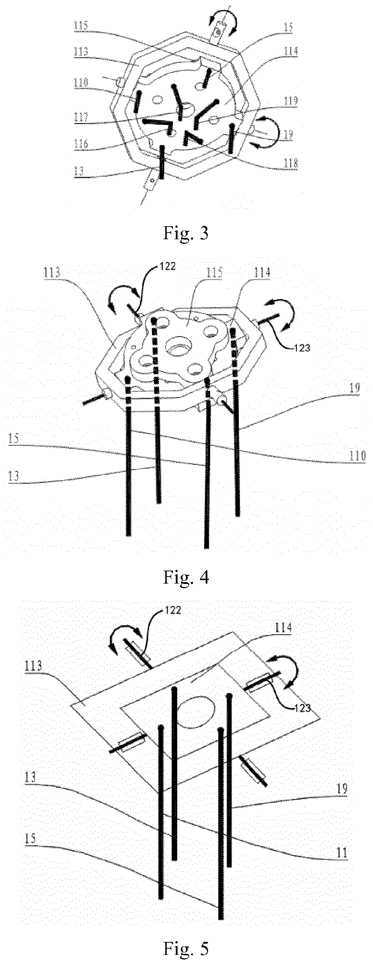

Referring to FIGs. 3, 4, and 5, a fixed point formed by a set of driving wires on the inner rotating member 114 is located on the first rotating shaft 122, and a fixed point formed by the other set of driving wires on the inner rotating member 114 is located at the second rotating shaft 123, and four fixed points formed by the four driving wires on the inner rotating member 114 are sequentially connected to form a first parallelogram, and an intersection point of diagonal lines of the first parallelogram is the first intersection point. Four fixed points formed by four output wires on the inner rotating member 114 are sequentially connected to form a second parallelogram, and an intersection point of diagonal lines of the second parallelogram is also the first intersection point. Preferably, the second parallelogram is a rectangle or a square, and the side of the second parallelogram is parallel to the first rotating shaft or the second rotating shaft. The fixed point formed by the driving wire on the inner rotating member 114 does not coincide with the fixed point formed by the output wire on the inner rotating member 114.

Still referring to FIGs. 2 and 7, the two wire rotating bodies include a first wire rotating body 12 and a second wire rotating body 18. The first wire rotating body 12 and the second wire rotating body 18 are disposed on the pedestal 120 and located on opposite sides of the guide wheel mounting base 121. Preferably, the first wire rotating body 12 and the second wire rotating body 18 are symmetrically disposed on two sides of the first through hole, and a connecting line between the first wire rotating body 12 and the second wire rotating body 18 is deviated from a connecting line between the first support member and the second support member by a predetermined angle.

The first wire rotating body 12 is provided with at least one wire mounting hole, and the second wire rotating body 18 is provided with at least one wire mounting hole. The wire mounting hole on the first wire rotating body 12 is used for fixing the driving wire on the first wire rotating body 12, and the wire mounting hole on the second wire rotating body 18 is used for fixing the driving wire on the second wire rotating body 18, so that the driving wires can be wound on the corresponding wire rotating bodies. Preferably, each wire rotating body is provided with two wire mounting holes for respectively fixing the driving wires wound thereon. The first wire rotating body 12 includes a first driving spindle 11 configured to drive the first wire rotating body 12 to perform a rotation motion, and an axis of the first driving spindle 11 is perpendicular to the pedestal 120. The second wire rotating body 18 includes a second driving spindle 17 configured to drive the second wire rotating body 18 to perform a rotation motion, and an axis of the second driving spindle 17 is perpendicular to the pedestal 120.

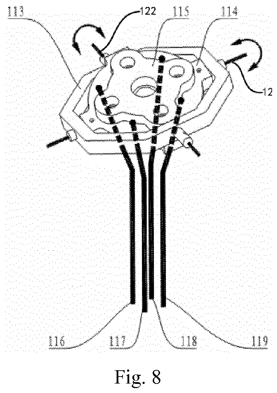

For a better understanding of the present invention, the following description will be made with reference to the contents shown in FIGs. 2 to 8.

In this embodiment, the driving wires and the output wires are wire ropes. The two sets of driving wires are respectively a first set of driving wires and a second set of driving wires. The four driving wires are a first wire rope 13, a second wire rope 15, a third wire rope 19, and a fourth wire rope 110. The first wire rope 13 and the second wire rope 15 constitute the first set of driving wires, and the third wire rope 19 and the fourth wire rope 110 constitute the second set of driving wires. The four output wires are a fifth wire rope 116, a sixth wire rope 117, a seventh wire rope 118, and an eighth wire rope 119, respectively. The first set of driving wires corresponds to the first wire rotating body 12, and the second set of driving wires corresponds to the second wire rotating body 18. The two guide wheel assemblies are a first guide wheel assembly and a second guide wheel assembly, respectively. The four guide wheels are a first guide wheel 16, a second guide wheel 14, a third guide wheel 111, and a fourth guide wheel 112. The first guide wheel 16 and the second guide wheel 14 constitute the first guide wheel assembly, and the third guide wheel 111 and the fourth guide wheel 112 constitute the second guide wheel assembly. The first guide wheel assembly corresponds to the first set of driving wires, and the second guide wheel assembly corresponds to the second set of driving wires.

The correspondence between the driving wire and the wire rotating body and the guide wheel in this embodiment is merely disclose the technical solution of the present invention more intuitively and highlight the beneficial effects of the present invention, and should not be deemed to limit the technical solution of the present invention.

### 1) For two sets of driving wires

One end of the first wire rope 13 is fixed on the inner rotating member 114 to form a first fixed point. The body of the first wire rope 13 extends downward, and after being steered by the first guide wheel 16, the other end thereof is wound and fixed on the first wire mounting hole of the first wire rotating body 12. One end of the second wire rope 15 is fixed to the inner rotating member 114 via the wire splint 115 to form a second fixed point. The body of the second wire rope 15 extends downward, after being steered by the second guide wheel 14, the other end thereof is wound and fixed on the second wire mounting hole of the first wire rotating body 12 in a direction opposite to the first wire rope 13. The first fixed point and the second fixed point are symmetrical about the first intersection point, and further, the first fixed point and the second fixed point are located on the first rotating shaft 122. When the first wire rotating body 12 rotates, since the winding directions of the first wire rope 13 and the second wire rope 15 on the first wire rotating body 12 are opposite, the first wire rope 13 and the second wire rope 15 move in opposite directions, so that the inner rotating member 114 is driven to be deflected about the second rotating shaft 123.

And the principle of the first set of driving wires for driving the inner rotating member 114 to rotate about the second rotating shaft 123 is as follows.

For example, the first wire rotating body 12 is rotated counterclockwise (as viewed from the side of the pedestal 120 on which the overturning platform assembly is located), the second wire rotating body 18 is stationary. The first wire rope 13 is wound on the first wire rotating body 12 counterclockwise, and the second wire rope 15 is wound on the first wire rotating body 12 clockwise. During the counterclockwise rotation movement of the first wire rotating body 12, the first wire rotating body 12 drives the first wire rope 13 to move downward, so as to further drive the portion of the inner rotating part 114 connected to the first wire rope 13 to move downward. On the other hand, the number of turns of the first wire rope 13 on the first wire rotating body 12 is increasing, and the number of turns of the second wire rope 15 on the first wire rotating body 12 is decreasing. At this time, the body of the first wire rope 13 between the first fixed point and the first wire rotating body 12 is becoming increasingly short, and the body of the second wire rope 15 between the second fixed point and the first wire rotating body 12 is becoming increasingly long. As this time, the inner rotating member 114 is rotated about the second rotating shaft 123 counterclockwise (as viewed from one side of the first wire rotating body 12, that is, the inner rotating member 114 is driven by the first set of driving wires to be deflected about the second rotating shaft 123 from the outside of the principal plane to the inside of the principal plane).

One end of the third wire rope 19 is fixed on the inner rotating member 114 to form a third fixed point. The body of the third wire rope 19 extends downward, and after being steered by the third guide wheel 111, the other end thereof is wound and fixed on the third wire mounting hole of the second wire rotating body 18. One end of the fourth wire rope 110 is fixed to the inner rotating member 114 to form a fourth fixed point. The body of the fourth wire rope 110 extends downward, after being steered by the first guide wheel 16, the other end thereof is wound and fixed on the fourth wire mounting hole of the second wire rotating body 18 in a direction opposite to the third wire rope 19. The third fixed point and the fourth fixed point are symmetrical about the first intersection point, and further, the third fixed point and the fourth fixed point are located on the second rotating shaft 123. When the second wire rotating body 18 rotates, since the winding directions of the third wire rope 19 and the fourth wire rope 110 on the second wire rotating body 18 are opposite, the third wire rope 19 and the fourth wire rope 110 move in opposite directions, so that the inner rotating member 114 is driven to be deflected about the second rotating shaft 122.

And the principle of the second set of driving wires for driving the inner rotating member 114 to rotate about the first rotating shaft 122 is as follows.

For example, the second wire rotating body 18 is rotated counterclockwise (as viewed from the side of the pedestal 120 on which the overturning platform assembly is located), the first wire rotating body 12 is stationary. The third wire rope 19 is wound on the second wire rotating body 18 counterclockwise, and the fourth wire rope 110 is wound on the second wire rotating body 18 clockwise. During the counterclockwise rotation movement of the second wire rotating body 18, the second wire rotating body 18 drives the third wire rope to move downward, so as to further drive the portion of the inner rotating part 114 connected to the third wire rope to move downward. On the other hand, the number of turns of the third wire rope 19 on the second wire rotating body 18 is increasing, and the number of turns of the fourth wire rope 110 on the second wire rotating body 18 is decreasing. At this time, the body of the third wire rope 19 between the third fixed point and the second wire rotating body 18 is becoming increasingly short, and the body of the fourth wire rope 110 between the fourth fixed point and the second wire rotating body 18 is becoming increasingly long. As this time, the inner rotating member 114 is deflected about the first rotating shaft 122 from the outside of the principal plane to the inside of the principal plane. As this time, the inner rotating member 114 is deflected about the second rotating shaft 123 (as viewed from one side of the second wire rotating body 18, the inner rotating member 114 is driven by the second set of driving wires to be deflected about the first rotating shaft 122 from the inside of the principal plane to the outside of the principal plane).

### 2) For four output wires

The fifth wire rope 116, the sixth wire rope 117, the seventh wire rope 118 and the eighth wire rope 119 respectively form a fifth fixed point, a sixth fixed point, a seventh fixed point and an eighth fixed point on the inner rotating member 114. The fifth fixed point and the eighth fixed point are symmetrical about the first intersection point, and the sixth fixed point and the seventh fixed point are symmetrical about the first intersection point. Furthermore, a straight line passing through the fifth fixed point and the sixth fixed point and a straight line passing through the seventh fixed point and the eighth fixed point are parallel to the first rotating shaft 122. A straight line passing through the fifth fixed point and the seventh fixed point and a straight line passing through the sixth fixed point and the eighth fixed point are parallel to the second rotating shaft 123. According to such configuration, the amount of change of all the output wires is substantially the same when the inner rotating member 114 is rotated about a rotating shaft (the first rotating shaft or the second rotating shaft), so that the movement condition of the end instrument assembly is improved.

And the principle that four output wires together drive the end instrument assembly 3 to achieve two degrees of freedom motion is as follows.

For example, the first wire rotating body 12 is rotated counterclockwise, the second wire rotating body 18 is stationary. The first wire rope 13 is wound on the first wire rotating body 12 counterclockwise, and the second wire rope 15 is wound on the first wire rotating body 12 clockwise. The first wire rope 13 and the second wire rope 15 are driven to move in opposite directions while the first wire rotating body 12 rotates (that is, the first wire rope 13 moves close to the first wire rotating body 12, and the second wire rope 15 moves far from the first wire rotating body 12), so that the inner rotating member 114 in the overturning platform assembly is rotated about the second rotating shaft 123 counterclockwise (as viewed from one side of the first wire rotating body 12, that is, the inner rotating member 114 is driven by the first set of driving wires to be deflected about the second rotating shaft 123 from the outside of the principal plane to the inside of the principal plane). Since the length of the output wires connected between the inner rotating member 114 and the end instrument assembly 3 is constant, during the deflection of the inner rotating member 114, the sixth wire rope 117 and the eighth wire rope 119 are driven to move together distant from the end instrument assembly 3. Meanwhile, the fifth wire rope 116 and the seventh wire rope 118 are driven to move together close to the end instrument assembly 3. On the contrary, for example, the first wire rotating body 12 rotates clockwise, the second wire rotating body 18 is stationary. The first wire rope 13 is wound on the first wire rotating body 12 counterclockwise, and the second wire rope 15 is wound on the first wire rotating body 12 clockwise. The first wire rope 13 and the second wire rope 15 are driven to move in opposite directions while the first wire rotating body 12 rotates (that is, the first wire rope 13 moves close to the first wire rotating body 12, and the second wire rope 15 moves distant from the first wire rotating body 12), so that the inner rotating member 114 in the overturning platform assembly is rotated about the second rotating shaft 123 clockwise (as viewed from one side of the first wire rotating body 12). Since the length of the output wires connected between the inner rotating member 114 and the end instrument assembly 3 is constant, the sixth wire rope 117 and the eighth wire rope 119 move together close to the end instrument assembly 3. Meanwhile, the fifth wire rope 116 and the seventh wire rope 118 move together distant from the end instrument assembly 3. Thus, the end instrument assembly 3 is driven by the four output wires to rotate, so as to achieve the motion of the end instrument assembly 3 in the first degree of freedom.

For example, the second wire rotating body 18 is rotated counterclockwise, the first wire rotating body 12 is stationary. The third wire rope 19 is wound on the second wire rotating body 18 counterclockwise, and the fourth wire rope 110 is wound on the second wire rotating body 18 clockwise. The third wire rope 19 and the fourth wire rope 110 are driven to move in opposite directions while the second wire rotating body 18 rotates (that is, the third wire rope 19 moves close to the first wire rotating body 12, and the fourth wire rope 110 moves distant from the first wire rotating body 12), so that the inner rotating member 114 in the overturning platform assembly is rotated about the first rotating shaft 122 clockwise (as viewed from one side close to the principal plane). Since the length of the output wires connected between the inner rotating member 114 and the end instrument assembly 3 is constant, during the deflection of the inner rotating member 114, the fifth wire rope 116 and the sixth wire rope 117 are driven to move together distant from the end instrument assembly 3. Meanwhile, the seventh wire rope 118 and the eighth wire rope 119 are driven to move together close to the end instrument assembly 3. On the contrary, for example, the second wire rotating body 18 is rotated clockwise, the first wire rotating body 12 is stationary. The third wire rope 19 is wound on the second wire rotating body 18 counterclockwise, and the fourth wire rope 110 is wound on the second wire rotating body 18 clockwise. The third wire rope 19 and the fourth wire rope 110 are driven to move in opposite directions while the second wire rotating body 18 rotates (that is, the third wire rope 19 moves distant from the first wire rotating body 12, and the fourth wire rope 110 moves close to the first wire rotating body 12), so that the inner rotating member 114 in the overturning platform assembly 3 is rotated about the first rotating shaft 122 counterclockwise (as viewed from one side close to the principal plane). Since the length of the output wires connected between the inner rotating member 114 and the end instrument assembly 3 is constant, during the deflection of the inner rotating member 114, the fifth wire rope 116 and the sixth wire rope 117 are driven to move together close to the end instrument assembly 3. Meanwhile, the seventh wire rope 118 and the eighth wire rope 119 are driven to move together distant from the end instrument assembly 3. Thus, the end instrument assembly 3 is driven by the four output wires to rotate, so as to achieve the motion of the end instrument assembly 3 in the second degree of freedom.

The above embodiment only illustrates the case where one of the two wire rotating bodies rotates, and similarly, the case where the two wire rotating bodies rotate can achieve any motion of the end instrument assembly 3 in two degrees of freedom, so that the end instrument assembly 3 simultaneously achieves multiple degrees of freedom motion.

In this embodiment, the positions of the first fixed point, the second fixed point, the third fixed point, and the fourth fixed point are configured as: the inner rotating member 114 in the overturning platform assembly is driven to rotate only about a rotating shaft when the first wire rope 13, and the second wire rope 15 move or the third wire rope 19, and the fourth wire rope 110 move. However, those skilled in the art know that the foregoing configuration is a preferred solution, as long as the first fixed point, the second fixed point, the third fixed point, and the fourth fixed point satisfy the conditions that the first fixed point and the second fixed point are symmetrical about the first intersection point, the third fixed point and the fourth fixed point are symmetrical about the first intersection point, and the connecting line between the first fixed point and the second fixed point does not overlap with the connecting line between the third fixed point and the fourth fixed point (that is, the first fixed point, the second fixed point, the third fixed point, and the fourth fixed point are sequentially connected to form a parallelogram). At this time, the inner rotating member 114 is driven to rotate about the two rotating shafts when the first wire rope 13, and the second wire rope 15 move or the third wire rope 19, and the fourth wire rope 110 move, and the amount of compensation movement of the fifth wire rope 116, the sixth wire rope 117, the seventh wire rope 118, and the eighth wire rope 119 can be obtained by calculation, to achieve the desired posture adjustment of the end instrument assembly 3. Similarly, in this embodiment, the fifth fixed point, the sixth fixed point, the seventh fixed point, and the eighth fixed point are configured as: the straight line passing through the fifth fixed point and the sixth fixed point and the straight line passing through the seventh fixed point and the eighth fixed point are parallel to the first rotating shaft 122. The straight line passing through the fifth fixed point and the seventh fixed point and the straight line passing through the sixth fixed point and the eighth fixed point are parallel to the second rotating shaft 123, which is also a preferred technical solution. With such configuration, the amount of movement of all the output wires is substantially the same when the inner rotating member 114 is rotated about a rotating shaft. Those skilled in the art can also achieve the above technical effects by means of a compensating member. For example, an overturning platform assembly corresponding to the overturning platform assembly in the instrument set is provided at the front end of the end instrument assembly 3 as a compensating member. In addition, some end instrument assemblies 3 also require that the amount of movement in the output wires is unequal to meet the particular requirements such as unequal deflection angles.

In conclusion, the above embodiments are helpful to those skilled in the art to further understand the present invention, but do not limit the present invention in any way. The invention is as defined by the appended claims.

In conclusion, in the instrument set and the surgical instrument provided by the present invention, the instrument set includes an overturning platform assembly, two sets of driving wires, two wire rotating bodies, and four output wires. Each set of driving wires includes two driving wires, two fixed points formed by each set of driving wires on an internal rotating component are symmetrical about a first intersection point, four fixed points formed by four driving wires on the internal rotating component are sequentially connected to form a first parallelogram, the intersection point of diagonal lines of the first parallelogram is the first intersection point, the other end of each of the two driving wires in each set of driving wires is wound on the corresponding wire rotating body in the opposite direction, and therefore the overturning platform assembly is driven to move by the four driving wires, and then motion of the overturning platform assembly is transferred to an end instrument assembly through the output wires, so that multi-direction and multi-degree-of-freedom motion of the end instrument assembly is achieved, thereby simplifying the design of the instrument set, reducing the volume of the instrument set, and increasing the space utilization of the instrument set.

## Claims

1. An apparatus (1) for controlling movement of a surgical instrument, comprising:
a pedestal (120);
an overturning platform assembly disposed above the pedestal (120) by a support member, wherein the overturning platform assembly comprises an inner rotating member (114), an outer rotating member (113), a first rotating shaft (122), and a second rotating shaft (123); the outer rotating member (113) is disposed on an outer side of the inner rotating member (114) and is connected to the inner rotating member (114) via the second rotating shaft (123); the outer rotating member (113) is able to rotate about an axis of the first rotating shaft (122), the inner rotating member (114) is able to rotate about an axis of the second rotating shaft (123), and the axis of the first rotating shaft (122) is intersected with the axis of the second rotating shaft (123) to form a first intersection point;
two sets of driving wires, each set of driving wires comprising two driving wires (13, 15, 19, 110);
two wire rotating bodies (12, 18) disposed on the pedestal (120) and configured to drive the driving wires;
each of the two sets of driving wires corresponds to one of the wire rotating bodies; one end of each of the two driving wires in each set of driving wires is fixed on the inner rotating member (114) and forms a fixation point; two fixation points formed by each set of driving wires are symmetrical about the first intersection point; four fixation points formed by the two sets of driving wires on the inner rotating member (114) are sequentially connected to form a first parallelogram, and an intersection point of diagonal lines of the first parallelogram coincides with the first intersection point; and the other end of each of the two driving wires in each set of driving wires is wound on a corresponding one of the wire rotating bodies in a direction opposite to the fixation direction of the one end of each of the two driving wires in each set of driving wires; and
four output wires (116, 117, 118, 119), wherein one end of each output wire is fixed on the inner rotating member (114) and forms a fixation point; four fixation points formed by the four output wires on the inner rotating member (114) are sequentially connected to form a second parallelogram, and an intersection point of diagonal lines of the second parallelogram coincides with the first intersection point; and the other end of each output wire extends through a first through hole formed on the pedestal (120).

2. The apparatus (1) for controlling movement of a surgical instrument according to claim 1, wherein the axis of the first rotating shaft (122) and the axis of the second rotating shaft (123) are perpendicular to each other .

3. The apparatus (1) for controlling movement of a surgical instrument according to claim 1 or 2, wherein the fixation points formed by one set of driving wires on the inner rotating member (114) are located on the axis of the first rotating shaft (122), and the fixation points formed by the other set of driving wires on the inner rotating member (114) are located on the axis of the second rotating shaft (123).

4. The apparatus (1) for controlling movement of a surgical instrument according to claim 1 or 2, wherein the second parallelogram is a rectangle or a square.

5. The apparatus (1) for controlling movement of a surgical instrument according to claim 1, further comprising two guide wheel assemblies, wherein each guide wheel assembly corresponds to one set of driving wires for changing an extension direction of the corresponding driving wires.

6. The apparatus (1) for controlling movement of a surgical instrument according to claim 5, wherein each guide wheel assembly comprises two guide wheels (14, 16, 111, 112), each guide wheel corresponds to one of the driving wires, and each driving wire forms two tangent points with the corresponding guide wheel, wherein one of the tangent points distant from the overturning platform assembly is a second tangent point, and the other one of the tangent points close to the overturning platform assembly is a first tangent point, and a portion of each driving wire between the first tangent point and the fixation point is perpendicular to the pedestal (120).

7. The apparatus (1) for controlling movement of a surgical instrument according to claim 5, further comprising a guide wheel mounting base (121) disposed on the pedestal (120), wherein the two guide wheel assemblies are disposed on the guide wheel mounting base (121).

8. The apparatus (1) for controlling movement of a surgical instrument according to claim 7, wherein a second through hole is formed on the guide wheel mounting base (121), and wherein the first through hole and the second through hole are concentric to each other and together constitute an extension channel for the four output wires.

9. The apparatus (1) for controlling movement of a surgical instrument according to claim 1, further comprising a wire splint (115) disposed on the inner rotating member (114) and configured to clamp and secure one end of the driving wire and/or one end of the output wire to the inner rotating member (114).

10. The apparatus (1) for controlling movement of a surgical instrument according to claim 1, wherein the support member comprises a first support member and a second support member, wherein the first support member and the second support member are disposed on the pedestal (120) and located on opposite sides of the second rotating shaft (123), and wherein the first support member and the second support member are rotatably connected to the outer rotating member (113) via the first rotating shaft (122) for supporting the overturning platform assembly.

11. The apparatus (1) for controlling movement of a surgical instrument according to claim 7, wherein the two wire rotating bodies comprise a first wire rotating body (12) and a second wire rotating body (18), and the first wire rotating body (12) and the second wire rotating body (18) are disposed on the pedestal (120) and located on opposite sides of the guide wheel mounting base (121).

12. The apparatus (1) for controlling movement of a surgical instrument according to claim 11, wherein the first wire rotating body (12) is provided with a wire mounting hole for fixing a corresponding driving wire on the first wire rotating body (12), and the second wire rotating body (18) is provided with a wire mounting hole for fixing a corresponding driving wire on the second wire rotating body (18).

13. The apparatus (1) for controlling movement of a surgical instrument according to claim 11, wherein the first wire rotating body (12) comprises a first driving spindle (11) configured to drive the first wire rotating body (12) to perform a rotation motion, and the second wire rotating body (18) comprises a second driving spindle (17) configured to drive the second wire rotating body (18) to perform a rotation motion.

14. A surgical instrument, comprising the apparatus (1) for controlling movement of a surgical instrument according to any one of claims 1-13, an instrument tube (2), and an end instrument assembly (3), wherein the apparatus (1) for controlling movement of a surgical instrument is connected to the end instrument assembly (3) via the instrument tube (2) and controls movement of the end instrument assembly (3).

15. The surgical instrument according to claim 14, wherein the instrument tube (2) has a lumen, and four output wires of the apparatus (1) for controlling movement of a surgical instrument are connected to the end instrument assembly (3) through the lumen of the instrument tube (2).

## Patentansprüche

1. Vorrichtung (1) zur Steuerung einer Bewegung eines Operationsinstruments, umfassend:
einen Sockel (120);
eine Kippplattformanordnung, die über dem Sockel (120) durch ein Stützelement angeordnet ist, wobei die Kippplattformanordnung einen inneren Drehkörper (114), einen äußeren Drehkörper (113), einen ersten Drehschaft (122) und einen zweiten Drehschaft (123) umfasst; wobei der äußere Drehkörper (113) an einer Außenseite des inneren Drehkörpers (114) angeordnet ist und mit dem inneren Drehkörper (114) über den zweiten Drehschaft (123) verbunden ist; wobei der äußere Drehkörper (113) um eine Achse des ersten Drehschafts (122) drehbar ist, wobei der innere Drehkörper (114) um eine Achse des zweiten Drehschafts (123) drehbar ist, und wobei die Achse des ersten Drehschafts (122) mit der Achse des zweiten Drehschafts (123) geschnitten wird, um einen ersten Schnittpunkt zu bilden;
zwei Sätze von Antriebsdrähten, wobei jeder Satz von Antriebsdrähten zwei Antriebsdrähte (13, 15, 19, 110) umfasst;
zwei Drahtdrehkörper (12, 18), die auf dem Sockel (120) angeordnet sind und ausgebildet sind, die Antriebsdrähte anzutreiben;
wobei jeder der zwei Sätze von Antriebsdrähten einem der Drahtdrehkörper entspricht; wobei ein Ende von jedem der zwei Antriebsdrähte in jedem Satz von Antriebsdrähten an dem inneren Drehkörper (114) befestigt ist und einen Befestigungspunkt bildet; wobei zwei Befestigungspunkte, die durch jeden Satz von Antriebsdrähten gebildet werden, symmetrisch um den ersten Schnittpunkt sind; wobei vier Befestigungspunkte, die durch die zwei Sätze von Antriebsdrähten auf dem inneren Drehkörper (114) gebildet werden, sequentiell verbunden sind, um ein erstes Parallelogramm zu bilden, und wobei ein Schnittpunkt von diagonalen Linien des ersten Parallelogramms mit dem ersten Schnittpunkt zusammenfällt; und wobei das andere Ende von jedem der zwei Antriebsdrähte in jedem Satz von Antriebsdrähten auf einem der entsprechenden Drahtdrehkörper gewickelt ist und zwar in einer Richtung entgegengesetzt zur Befestigungsrichtung des einen Endes von jedem der zwei Antriebsdrähte in jedem Satz von Antriebsdrähten; und
vier Ausgangsdrähte (116, 117, 118, 119), wobei ein Ende jedes Ausgangsdrahts an dem inneren Drehkörper (114) befestigt ist und einen Befestigungspunkt bildet; wobei vier Befestigungspunkte, die durch die vier Ausgangsdrähte auf dem inneren Drehkörper (114) gebildet werden, sequentiell verbunden sind, um ein zweites Parallelogramm zu bilden, und ein Schnittpunkt von diagonalen Linien des zweiten Parallelogramms mit dem ersten Schnittpunkt zusammenfällt; und wobei das andere Ende von jedem Ausgangsdraht sich durch ein erstes Durchgangsloch erstreckt, das auf dem Sockel (120) gebildet ist.

2. Vorrichtung (1) zur Steuerung einer Bewegung eines Operationsinstruments nach Anspruch 1, wobei die Achse des ersten Drehschafts (122) und die Achse des zweiten Drehschafts (123) senkrecht zueinander sind.

3. Vorrichtung (1) zur Steuerung einer Bewegung eines Operationsinstruments nach Anspruch 1 oder 2, wobei die Befestigungspunkte, die durch einen Satz von Antriebsdrähten auf dem inneren Drehkörper (114) gebildet sind, sich auf der Achse des ersten Drehschafts (122) befinden, und wobei die Befestigungspunkte, die durch den anderen Satz von Antriebsdrähten auf dem inneren Drehkörper (114) gebildet sind, sich auf der Achse des zweiten Drehschafts (123) befinden.

4. Vorrichtung (1) zur Steuerung einer Bewegung eines Operationsinstruments nach Anspruch 1 oder 2, wobei das zweite Parallelogramm ein Rechteck oder ein Quadrat ist.

5. Vorrichtung (1) zur Steuerung einer Bewegung eines Operationsinstruments nach Anspruch 1, ferner umfassend zwei Führungsradanordnungen, wobei jede Führungsradanordnung einem Satz von Antriebsdrähten zum Ändern einer Erstreckungsrichtung der entsprechenden Antriebsdrähte entspricht.

6. Vorrichtung (1) zur Steuerung einer Bewegung eines Operationsinstruments nach Anspruch 5, wobei jede Führungsradanordnung zwei Führungsräder (14, 16, 111, 112) umfasst, wobei jedes Führungsrad einem der Antriebsdrähte entspricht, und wobei jeder Antriebsdraht zwei Tangentenpunkte mit dem entsprechenden Führungsrad bildet, wobei einer der Tangentenpunkte, der von der Kippplattformanordnung entfernt ist, ein zweiter Tangentenpunkt ist, und der andere der Tangentenpunkte, der der Kippplattformanordnung nahe ist, ein erster Tangentenpunkt ist, und wobei ein Abschnitt jedes Antriebsdrahts zwischen dem ersten Tangentenpunkt und dem Befestigungspunkt senkrecht zum Sockel (120) ist.

7. Vorrichtung (1) zur Steuerung einer Bewegung eines Operationsinstruments nach Anspruch 5, ferner umfassend eine Führungsradbefestigungsbasis (121), die auf dem Sockel (120) angeordnet ist, wobei die zwei Führungsradanordnungen auf der Führungsradbefestigungsbasis (121) angeordnet sind.

8. Vorrichtung (1) zur Steuerung einer Bewegung eines Operationsinstruments nach Anspruch 7, wobei ein zweites Durchgangsloch an der Führungsradbefestigungsbasis (121) gebildet ist, und wobei das erste Durchgangsloch und das zweite Durchgangsloch konzentrisch zueinander sind und zusammen einen Verlängerungskanal für die vier Ausgangsdrähte bilden.

9. Vorrichtung (1) zur Steuerung einer Bewegung eines Operationsinstruments nach Anspruch 1, ferner umfassend eine Drahtschiene (115), die an dem inneren Drehkörper (114) angeordnet ist und ausgebildet ist, ein Ende des Antriebsdrahts und/oder ein Ende des Ausgangsdrahts an den inneren Drehkörper (114) zu klemmen und zu befestigen.

10. Vorrichtung (1) zur Steuerung einer Bewegung eines Operationsinstruments nach Anspruch 1, wobei das Stützelement ein erstes Stützelement und ein zweites Stützelement umfasst, wobei das erste Stützelement und das zweite Stützelement auf dem Sockel (120) angeordnet sind und sich an gegenüberliegenden Seiten des zweiten Drehschafts (123) befinden, und wobei das erste Stützelement und das zweite Stützelement über den erste Drehschaft (122) zum Stützen der Kippplattformanordnung drehbar mit dem äußeren Drehkörper (113) verbunden sind.

11. Vorrichtung (1) zur Steuerung einer Bewegung eines Operationsinstruments nach Anspruch 7, wobei die zwei Drahtdrehkörper einen ersten Drahtdrehkörper (12) und einen zweiten Drahtdrehkörper (18) umfassen, und wobei der erste Drahtdrehkörper (12) und der zweite Drahtdrehkörper (18) auf dem Sockel (120) angeordnet sind und sich auf gegenüberliegenden Seiten der Führungsradbefestigungsbasis (121) befinden.

12. Vorrichtung (1) zur Steuerung einer Bewegung eines Operationsinstruments nach Anspruch 11, wobei der erste Drahtdrehkörper (12) mit einem Drahtmontageloch zur Befestigung eines entsprechenden Antriebsdrahts an dem ersten Drahtdrehkörper (12) versehen ist, und wobei der zweite Drahtdrehkörper (18) mit einem Drahtmontageloch zur Befestigung eines entsprechenden Antriebsdrahts an dem zweiten Drahtdrehkörper (18) versehen ist.

13. Vorrichtung (1) zur Steuerung einer Bewegung eines Operationsinstruments nach Anspruch 11, wobei der erste Drahtdrehkörper (12) eine erste Antriebsspindel (11) umfasst, die ausgebildet ist, den ersten Drahtdrehkörper (12) anzutreiben eine Drehbewegung auszuführen, und wobei der zweite Drahtdrehkörper (18) eine zweite Antriebsspindel (17) umfasst, die ausgebildet ist, den zweiten Drahtdrehkörper (18) anzutreiben eine Drehbewegung auszuführen.

14. Operationsinstrument, umfassend die Vorrichtung (1) zur Steuerung einer Bewegung eines Operationsinstruments nach einem der Ansprüche 1-13, ein Instrumentenrohr (2) und eine Endinstrumentenanordnung (3), wobei die Vorrichtung (1) zur Bewegungssteuerung eines Operationsinstruments über das Instrumentenrohr (2) mit der Endinstrumentenanordnung (3) verbunden ist und die Bewegung der Endinstrumentenanordnung (3) steuert.

15. Operationsinstrument nach Anspruch 14, wobei das Instrumentenrohr (2) ein Lumen aufweist, und wobei vier Ausgangsdrähte der Vorrichtung (1) zur Steuerung einer Bewegung eines Operationsinstruments mit der Endinstrumentenanordnung (3) durch das Lumen des Instrumentenrohrs (2) verbunden sind.

## Revendications

1. Appareil (1) pour commander le mouvement d'un instrument chirurgical, comprenant :
un socle (120) ;
un ensemble plate-forme de retournement disposé au-dessus du socle (120) par un élément de support, l'ensemble plate-forme de retournement comprenant un élément rotatif interne (114), un élément rotatif externe (113), un premier arbre rotatif (122) et un second arbre rotatif (123) ; l'élément rotatif externe (113) étant disposé sur un côté extérieur de l'élément rotatif interne (114) et étant relié à l'élément rotatif interne (114) par l'intermédiaire du second arbre rotatif (123) ; l'élément rotatif externe (113) étant apte à tourner autour d'un axe du premier arbre rotatif (122), l'élément rotatif interne (114) étant apte à tourner autour d'un axe du second arbre rotatif (123), et l'axe du premier arbre rotatif (122) croisant l'axe du second arbre rotatif (123) pour former un premier point d'intersection ;
deux groupes de fils d'entraînement, chaque groupe de fils d'entraînement comprenant deux fils d'entraînement (13, 15, 19, 110) ;
deux corps rotatifs de fil (12, 18) disposés sur le socle (120) et configurés pour entraîner les fils d'entraînement ;
chacun des deux groupes de fils d'entraînement correspondant à l'un des corps rotatifs de fil ; une extrémité de chacun des deux fils d'entraînement dans chaque groupe de fils d'entraînement étant fixée sur l'élément rotatif interne (114) et formant un point de fixation ; deux points de fixation formés par chaque groupe de fils d'entraînement étant symétriques autour du premier point d'intersection ; quatre points de fixation formés par les deux groupes de fils d'entraînement sur l'élément rotatif interne (114) étant reliés séquentiellement pour former un premier parallélogramme, et un point d'intersection des lignes diagonales du premier parallélogramme coïncidant avec le premier point d'intersection ; et l'autre extrémité de chacun des deux fils d'entraînement de chaque groupe de fils d'entraînement étant enroulée sur un corps correspondant des corps rotatifs de fil dans une direction opposée à la direction de fixation de la première extrémité de chacun des deux fils d'entraînement de chaque groupe de fils d'entraînement ; et
quatre fils de sortie (116, 117, 118, 119), une extrémité de chaque fil de sortie étant fixée sur l'élément rotatif interne (114) et formant un point de fixation ; quatre points de fixation formés par les quatre fils de sortie sur l'élément rotatif interne (114) étant reliés séquentiellement pour former un second parallélogramme, et un point d'intersection des lignes diagonales du second parallélogramme coïncidant avec le premier point d'intersection ; et l'autre extrémité de chaque fil de sortie s'étendant à travers un premier trou traversant formé sur le socle (120).

2. Appareil (1) pour commander le mouvement d'un instrument chirurgical selon la revendication 1, l'axe du premier arbre rotatif (122) et l'axe du second arbre rotatif (123) étant perpendiculaires l'un à l'autre.

3. Appareil (1) pour commander le mouvement d'un instrument chirurgical selon la revendication 1 ou 2, les points de fixation formés par un groupe de fils d'entraînement sur l'élément rotatif interne (114) étant situés sur l'axe du premier arbre rotatif (122), et les points de fixation formés par l'autre groupe de fils d'entraînement sur l'élément rotatif interne (114) étant situés sur l'axe du second arbre rotatif (123).

4. Appareil (1) pour commander le mouvement d'un instrument chirurgical selon la revendication 1 ou 2, le second parallélogramme étant un rectangle ou un carré.

5. Appareil (1) pour commander le mouvement d'un instrument chirurgical selon la revendication 1, comprenant en outre deux ensembles de roues de guidage, chaque ensemble de roues de guidage correspondant à un groupe de fils d'entraînement pour changer une direction d'extension des fils d'entraînement correspondants.

6. Appareil (1) pour commander le mouvement d'un instrument chirurgical selon la revendication 5, chaque ensemble de roue de guidage comprenant deux roues de guidage (14, 16, 111, 112), chaque roue de guidage correspondant à l'un des fils d'entraînement, et chaque fil d'entraînement formant deux points tangents avec la roue de guidage correspondante, l'un des points tangents distant de l'ensemble plate-forme de retournement étant un second point tangent, et l'autre des points tangents proche de l'ensemble plate-forme de retournement étant un premier point tangent, et une partie de chaque fil d'entraînement entre le premier point tangent et le point de fixation étant perpendiculaire au socle (120).

7. Appareil (1) pour commander le mouvement d'un instrument chirurgical selon la revendication 5, comprenant en outre un support de montage de roue de guidage (121) disposé sur le socle (120), les deux ensembles de roue de guidage étant disposés sur le support de montage de roue de guidage (121).

8. Appareil (1) pour commander le mouvement d'un instrument chirurgical selon la revendication 7, un second trou traversant étant formé sur le support de montage de roue de guidage (121), et le premier trou traversant et le second trou traversant étant concentriques l'un par rapport à l'autre et constituant ensemble un canal d'extension pour les quatre fils de sortie.

9. Appareil (1) pour commander le mouvement d'un instrument chirurgical selon la revendication 1, comprenant en outre une attelle à fil (115) disposée sur l'élément rotatif interne (114) et configurée pour serrer et fixer une extrémité du fil d'entraînement et/ou une extrémité du fil de sortie à l'élément rotatif interne (114).

10. Appareil (1) pour commander le mouvement d'un instrument chirurgical selon la revendication 1, l'élément de support comprenant un premier élément de support et un second élément de support, le premier élément de support et le second élément de support étant disposés sur le socle (120) et situés sur des côtés opposés du second arbre rotatif (123), et le premier élément de support et le second élément de support étant reliés de manière rotative à l'élément rotatif externe (113) par l'intermédiaire du premier arbre rotatif (122) pour supporter l'ensemble plate-forme de retournement.

11. Appareil (1) pour commander le mouvement d'un instrument chirurgical selon la revendication 7, les deux corps rotatifs de fil comprenant un premier corps rotatif de fil (12) et un second corps rotatif de fil (18), et le premier corps rotatif de fil (12) et le second corps rotatif de fil (18) étant disposés sur le socle (120) et situés sur des côtés opposés du support de montage de roue de guidage (121).

12. Appareil (1) pour commander le mouvement d'un instrument chirurgical selon la revendication 11, le premier corps rotatif de fil (12) étant muni d'un trou de montage de fil pour fixer un fil d'entraînement correspondant sur le premier corps rotatif de fil (12), et le second corps rotatif de fil (18) étant muni d'un trou de montage de fil pour fixer un fil d'entraînement correspondant sur le second corps rotatif de fil (18).

13. Appareil (1) pour commander le mouvement d'un instrument chirurgical selon la revendication 11, le premier corps rotatif de fil (12) comprenant une première broche d'entraînement (11) configurée pour entraîner le premier corps rotatif de fil (12) pour réaliser un mouvement de rotation, et le second corps rotatif de fil (18) comprenant une seconde broche d'entraînement (17) configurée pour entraîner le second corps rotatif de fil (18) pour réaliser un mouvement de rotation.

14. Instrument chirurgical, comprenant l'appareil (1) pour commander le mouvement d'un instrument chirurgical selon l'une quelconque des revendications 1 à 13, un tube d'instrument (2), et un ensemble instrument d'extrémité (3), l'appareil (1) pour commander le mouvement d'un instrument chirurgical étant relié à l'ensemble instrument d'extrémité (3) par l'intermédiaire du tube d'instrument (2) et commandant le mouvement de l'ensemble instrument d'extrémité (3).

15. Instrument chirurgical selon la revendication 14, le tube d'instrument (2) ayant une lumière, et quatre fils de sortie de l'appareil (1) pour commander le mouvement d'un instrument chirurgical étant reliés à l'ensemble instrument d'extrémité (3) au moyen de la lumière du tube d'instrument (2).
